(19) **Europäisches Patentamt**

**European Patent Office**

**Office européen des brevets**

(11) **EP 0 909 175 B1**

(12) **EUROPEAN PATENT SPECIFICATION**

(45) Date of publication and mention
of the grant of the patent:
**04.06.2003 Bulletin 2003/23**

(21) Application number: **97930181.9**

(22) Date of filing: **01.07.1997**

(51) Int Cl.⁷: **A61K 38/00**, A61K 38/04,
A61K 38/08, A61K 38/24,
A61K 47/00, A61K 47/16,
A61K 47/18, A61K 47/20,
A61K 38/09, A61K 47/02,
A61K 47/06

(86) International application number:
**PCT/US97/10815**

(87) International publication number:
**WO 98/000152 (08.01.1998 Gazette 1998/01)**

(54) **NON-AQUEOUS PROTIC PEPTIDE FORMULATIONS**

NICHT-WAESSRIGE PROTISCHE PEPTIDZUBEREITUNGEN

FORMULATIONS PROTIQUES NON AQUEUSES DE PEPTIDES

(84) Designated Contracting States:
**AT BE CH DE DK ES FI FR GB GR IE IT LI LU NL
PT SE**

(30) Priority: **03.07.1996 US 21129 P**

(43) Date of publication of application:
**21.04.1999 Bulletin 1999/16**

(73) Proprietor: **ALZA CORPORATION**
**Palo Alto California 94303-0802 (US)**

(72) Inventors:
• **ECKENHOFF, James, B.**
**deceased (US)**
• **STEVENSON, Cynthia, L.**
**Mountain View, CA 94040 (US)**
• **TAO, Sally, A.**
**San Jose, CA 95129 (US)**
• **PRESTRELSKI, Steven, J.**
**Mountain View, CA 94043 (US)**
• **WRIGHT, Jeremy, C.**
**Los Altos, CA 94024 (US)**
• **LEONARD, Joe**
**Cupertino, CA 95014 (US)**

(74) Representative: **Evans, David Charles**
**fJ CLEVELAND**
**40-43, Chancery Lane**
**London, WC2A 1JQ (GB)**

(56) References cited:
**EP-A- 0 312 052**     **EP-A- 0 510 731**
**WO-A-94/06452**     **WO-A-94/19020**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Description

## CROSS-REFERENCE TO RELATED APPLICATIONS

This application claims priority under 35 U.S.C. 119(e) to U.S. Application Serial No. 60/021,129 filed July 3, 1996, the disclosure of which is incorporated herein by reference.

## FIELD OF THE INVENTION

This invention relates to stable non-aqueous protic formulations of peptide compounds. In particular, formulations with high concentrations of peptide compounds are provided.

## BACKGROUND OF THE INVENTION

References:

The following references are referred to by numbers in brackets ([ ]) at the relevant portion of the specification.

1. Zoladex (goserelin acetate implant), Physician's Desk Reference, 50th Edition, pages 2858-2861 (1996).
2. U.S. Patent No. 3,914,412, issued October 21, 1975.
3. U.S. Patent No. 4,547,370, issued October 15, 1985.
4. U.S. Patent No. 4,661,472, issued April 28, 1987.
5 U.S. Patent No. 4,689,396, issued August 25, 1987.
6 U.S. Patent No. 4,851,385, issued July 25, 1989.
7. U.S. Patent No. 5,198,533, issued March 30, 1993.
8. U.S. Patent No. 5,480,868, issued January 2, 1996.
9. WO92/20711, published 26 November 1992.
10. WO95/00168, published 5 January 1995.
11. WO95/04540, published 16 February 1995.
12. "Stability of Gonadorelin and Triptorelin in Aqueous Solution", V.J. Helm, B.W. Muller, *Pharmaceutical* Research, 7/12, pages 1253-1256 (1990).
13. "New Degradation Product of Des-Gly$^{10}$-NH$_2$LH-RH-Ethylamide (Fertirelin) in Aqueous Solution", J. Okada, T. Seo, F. Kasahara, K. Takeda, S. Kondo, *J. of Pharmaceutical Sciences,* 80/2, pages 167-170(1991).
14. "Characterization of the Solution Degradation Product of Histrelin, a Gonadotropin Releasing Hormone (LHRH) Agonist", A.R. Oyler, R.E. Naldi, J.R. Lloyd, D.A. Graden, C.J. Shaw, M.L. Cotter, *J. of Pharmaceutical Sciences,* 80/3, pages 271-275 (1991).
15. "Parenteral Peptide Formulations: Chemical and Physical Properties of Native Luteinizing Hormone-Releasing Hormone (LHRH) and Hydrophobic Analogues in Aqueous Solution", M.F. Powell, L.M. Sanders, A. Rogerson, V. Si, *Pharmaceutical Research,* 8/10, pages 1258-1263 (1991).
16. "Degradation of the LHRH Analog Nafarelin Acetate in Aqueous Solution", D.M. Johnson, R.A. Pritchard, W. F. Taylor, D. Conley, G. Zuniga, K.G. McGreevy, *Intl. J. of Pharmaceutics,* 31, pages 125-129 (1986).
17. "Percutaneous Absorption Enhancement of Leuprolide", M.Y. Fu Lu, D. Lee, G.S. Rao, *Pharmaceutical Research,* 9/12, pages 1575-1576 (1992).
18. Lutrepulse (gonadorelin acetate for IV injection), Physician's Desk Reference, 50th Edition, pages 980-982 (1996).
19. Factrel (gonadorelin HCI for subcutaneous or IV injection), Physician's Desk Reference, 50th Edition, pages 2877-2878 (1996).
20. Lupron (leuprolide acetate for subcutaneous injection), Physician's Desk Reference, 50th Edition, pages 2555-2556 (1996).
21. Lupron depot (leuprolide acetate for depot suspension), Physician's Desk Reference, 50th Edition, pages 2556-2562 (1996).
22. "Pharmaceutical Manipulation of Leuprorelin Acetate to Improve Clinical Performance", H. Toguchi, *J. of Intl. Medical Research,* 18, pages 35-41 (1990).
23. "Long-Term Stability of Aqueous Solutions of Luteinizing Hormone-Releasing Hormone Assessed by an In-Vitro Bioassay and Liquid Chromatography", Y.F. Shi, R. J. Sherins, D. Brightwell, J.F. Gallelli, D. C. Chatterji, *J. of Pharmaceutical Sciences,* 73/6, pages 819-821 (1984).
24. "Peptide Liquid Crystals: Inverse Correlation of Kinetic Formation and Thermodynamic Stability in Aqueous Solution", M.F. Powell, J. Fleitman, L.M. Sanders, V.C. Si, *Pharmaceutical Research,* 11/9, pages 1352-1354 (1994).

25. "Solution Behavior of Leuprolide Acetate, an LHRH Agonist, as Determined by Circular Dichroism Spectroscopy", M.E. Powers, A. Adejei, M.Y. Fu Lu, M.C. Manning, *intl. J. of Pharmaceutics,* 108, pages 49-55 (1994).

26. "Preparation of Three-Month Depot Injectable Microspheres of Leuprorelin Acetate Using Biodegradable Polymers", *Pharmaceutical Research,* 11/8, pages 1143-1147 (1994).

Luteinizing hormone-releasing hormone (LHRH), also known as gonadotropin releasing hormone (GnRH), is a decapeptide with the structure:

pGlu-His-Trp-Ser-Tyr-Gly-Leu-Arg-Pro-Gly-NH$_2$.

It is secreted by the hypothalamus and binds to receptors on the pituitary gland, releasing luteinizing hormone (LH) and follicle stimulating hormone (FSH). LH and FSH stimulate the gonads to synthesize steroid hormones. Numerous analogs of LHRH are known, including peptides related to LHRH which act as agonists and those which act as antagonists. [1-15] LHRH analogs are known to be useful for treating hormone-dependent diseases such as prostate cancer, benign prostatomegaly, endometriosis, hysteromyoma, metrofibroma, precocious puberty, or mammary cancer and as contraceptives. [8] Sustained release administration is preferred for both agonist LHRH-related compounds, which reduce the number of available receptors after repeated administration so that the production of steroid hormones is suppressed, and antagonist LHRH-related compounds, which must be continually administered for persistent inhibition of endogenous LHRH. [8]

The sustained parenteral delivery of drugs, especially peptide drugs, provides many advantages. The use of implantable devices for sustained delivery of a wide variety of drugs or other beneficial agents is well known in the art. Typical devices are described, for example, in U.S. Patents Nos. 5,034,229; 5,057,318; and 5,110,596. The disclosure of each of these patents is incorporated herein by reference.

In general, oral bioavailability of peptides, including LHRH-related compounds, is low. [16-17]

Currently marketed formulations of LHRH, its analogs and related compounds which are used for parenteral injection are aqueous solutions which contain relatively low concentrations of LHRH-related compounds (0.05 to 5 mg/ml) and may also contain excipients such as mannitol or lactose. [18-20] Such formulations of LHRH-related compounds must either be stored refrigerated or may be stored at room temperature for short periods of time.

Available depot formulations of LHRH-related compounds administered for sustained release over a period of 1-3 months include a formulation comprised of 15% LHRH-related compound dispersed in a matrix of D,L-lactic and glycolic acids copolymer presented as a cylinder to be injected subcutaneously [1] and a formulation comprised of microparticles comprising a core of LHRH-related compound and gelatin surrounded by a shell of D,L-lactic and glycolic acids copolymer. These microparticles are suspended in a diluent for injection either subcutaneously or intramuscularly. [21, 26] These products must be stored at room temperature or lower. Aqueous formulations of LHRH-related compounds are known to exhibit both chemical and physical instability, as well as degradation after irradiation. [12-16, 22-25]

WO 94/06452 discloses protein suspensions, EP-A-0 510 731 discloses LHRH analog suspension aerosols and WO 94/19020 describes aqueous polypeptide solutions.

Formulations which have been shown to be stable ($t_{90}$ about five years) have been very low concentration (25 µg/ml) aqueous, buffered (10 mM, ionic strength of 0.15) solutions stored at temperatures no higher than room temperature (25°C). [15]

There is a need for stable formulations of peptides.

## SUMMARY OF THE INVENTION

The present invention provides stable non-aqueous formulations which are solutions of peptide compounds in non-aqueous protic solvents. In particular, formulations with concentrations of at least about 10% peptide are provided. These stable formulations may be stored at elevated temperatures (e.g., 37°C) for long periods of time and are especially useful in implantable delivery devices for long term delivery (e.g., 1-12 month or longer) of drug.

In one aspect, the invention provides a stable non-aqueous formulation according to claim 1 for administration to a patient, comprising:

a) at least one peptide compound; and
b) a solvent for the peptide compound, which solvent consists of at least one non-aqueous protic solvent in which said peptide compound has a solubility of at least 10% (w/w) and in which the peptide compound is dissolved,

wherein said formulation is stable at 37°C for at least 2 months, so that at least 65% chemically and physically stable peptide compound remains after two months at 37°C, of peptide compounds, said formulations comprising at least one peptide compound in at least one non-aqueous protic solvent.

In another aspect, the invention provides a method for preparing the stable non-aqueous formulation of claim 1 comprising providing at least one peptide compound and selecting a solvent for the peptide compound which solvent consists of at least one non-aqueous protic solvent in which said peptide compound has a solubility of at least 10% (w/w), and dissolving said peptide compound in the non-aqueous protic solvent.

In yet a further aspect, the invention can be used for treating a subject suffering from a condition which may be alleviated by administration of an peptide compound by administering to said subject an effective amount of a stable non-aqueous formulation comprising at least one peptide compound in at least one non-aqueous protic solvent

## BRIEF DESCRIPTION OF THE DRAWINGS

Figure 1 illustrates the stability of 40% leuprolide acetate solution in propylene glycol (PG) after two months at 80°C as measured by reverse phase HPLC (RP-HPLC).

Figure 2 shows RP-HPLC of the same sample as Figure 1 injected on size exclusion chromatography (SEC) depicting 3% dimer and trimer formation with no higher order aggregates detected.

Figure 3 presents the Arrhenius plot showing the loss of leuprolide from 40% solutions of leuprolide acetate in propylene glycol (PG).

Figure 4 illustrates the loss of leuprolide from a 40% leuprolide solution in PG over a period of four to six months at 37°C, 50°C, 65°C or 80°C.

Figure 5 illustrates the chemical and physical stability of a 40% leuprolide solution in PG after four months at 80°C.

Figure 6 illustrates the chemical stability of a 40% leuprolide acetate solution in PG after nine months at 37°C.

Figure 7 illustrates the chemical stability of a 40% leuprolide acetate solution in PG/acetate buffer (30:70) after one year at 37°C.

Figure 8 illustrates the physical stability of a 40% leuprolide acetate solution in PG/acetate buffer (30:70) after one year at 37°C.

Figure 9 illustrates the stability of a 40% leuprolide acetate solution in PG/water with preservatives (30:70) after six months at 60°C after irradiation.

Figure 10 illustrates the long term stability of a 40% leuprolide acetate solution in PG/water (30:70) over a six month period at 37°C after irradiation.

Figure 11 illustrates the stability of a 30% goserelin solution in PEG 600/acetate buffer (30:70) after 14 days at 80°C.

## DETAILED DESCRIPTION OF THE INVENTION

The present invention is drawn to the unexpected discovery that dissolving peptide compounds in non-aqueous protic solvents results in stable formulations. Previously known formulations of peptide compounds, which are dilute buffered aqueous solutions containing excipients such as EDTA or ascorbic acid which must be stored at low temperatures (4-25°C), form degradation products using degradation pathways such as acid/base catalyzed hydrolysis, deamidation, racemization and oxidation. In contrast, the presently claimed formulations stabilize peptide compounds at elevated temperatures (e.g., 37°C to 80°C) and at high concentrations (i.e., at least about 10%).

Standard peptide and protein formulations consist of dilute aqueous solutions. Two critical aspects of peptide formulation include solubilization and stabilization of the drug molecule.

Peptide solubilization under aqueous conditions is standard, because it mimics nature. However, solubilization under non-aqueous conditions is not known. We have found that peptide formulation is possible in non-aqueous protic solvents.

Peptide stability is usually achieved by varying one or more of the following: pH, buffer type, ionic strength, excipients (EDTA, ascorbic acid, etc.). For these formulations, degradation pathways requiring water (hydrolysis, deamidation, racemization) cannot be fully stabilized. In contrast, in the present invention, highly concentrated peptides formulated in non-aqueous solutions such as propylene glycol and polyethylene glycol were shown to be chemically and physically stable. Such solvents are considered non-aqueous protic solvents. Some non-aqueous protic solvents may function to decrease the rate of degradation because they do not have large dipole moments needed for the stabilization of rate determining steps.

The invention consists of using non-aqueous protic solvents such as propylene glycol and polyethylene glycols to stabilize highly concentrated peptide and protein formulations against both chemical and physical degradation. The discovery consists of the realization that use of propylene glycol or polyethylene glycols improves the overall solubility and stability of peptides in a wide range of formulation conditions, including high concentrations and elevated temperatures, thus making possible the delivery of peptides in implantable delivery devices that would not otherwise be feasible.

A. Definitions:

As used herein, the following terms have the following meanings:

The term "chemical stability" means that an acceptable percentage of degradation products produced by chemical pathways such as oxidation or hydrolysis is formed. In particular, a formulation is considered chemically stable if no more than about 20% breakdown products are formed after two months at 37°C.

The term "physical stability" means that an acceptable percentage of aggregates (e.g., dimers, trimers and larger forms) is formed. In particular, a formulation is considered physically stable if no more that about 15% aggregates are formed after two months at 37°C.

The term "stable formulation" means that at least about 65% chemically and physically stable peptide compound remains after two months at 37°C (or equivalent conditions at an elevated temperature). Particularly preferred formulations are those which retain at least about 80% chemically and physically stable compound under these conditions. Especially preferred stable formulations are those which do not exhibit degradation after sterilizing irradiation (e.g., gamma, beta or electron beam).

The terms " peptide" and/or "peptide compound" mean polymers of up to about 50 amino acid residues bound together by amide (CONH) linkages. Analogs, derivatives, agonists, antagonists and pharmaceutically acceptable salts of any of these are included in these terms. The terms also include peptides and/or peptide compounds which have D-amino acids, modified, derivatized or non-naturally occurring amino acids in the D- or L- configuration and/or peptomimetic units as part of their structure.

The term "LHRH-related compound" means luteinizing hormone releasing hormone (LHRH) and its analogs and pharmaceutically acceptable salts. Octa-, nona- and decapeptide LHRH agonists and antagonists are included in the term LHRH-related compounds, as is native LHRH. Particularly preferred LHRH-related compounds include LHRH, leuprolide, goserelin, nafarelin, and other known active agonists and antagonists. [1-21]

The term "high concentration" means at least about 10% (w/w) and up to the maximum solubility of the particular compound.

The term "excipient" means a more or less inert substance in a formulation which is added as a diluent or vehicle or to give form or consistency. Excipients are distinguished from solvents such as EtOH, which are used to dissolve drugs in formulations, from non-ionic surfactants such as Tween 20, which are used to solubilize drugs in formulations, and from preservatives such as benzyl alcohols and methyl or propyl parabens, which are used to prevent or inhibit microbial growth.

The term "non-aqueous protic solvent" means a non-aqueous solvent which contains hydrogen attached to oxygen or nitrogen so that it is able to form hydrogen bonds or donate a proton. Examples of non-aqueous protic solvents are polyethylene glycols (PEGs), propylene glycol (PG), polyvinylpyrrolidone (PVP), methoxypropylene glycol (MPEG), glycerol and glycofurol.

The term "polar aprotic solvent" means a polar solvent which does not contain acidic hydrogen and does not act as a hydrogen bond donor. Examples of polar aprotic solvents are dimethylsulfoxide (DMSO), dimethylformamide (DMF), hexamethylphosphorotriamide (HMPT), and n-methyl pyrrolidone.

B. Preparation of Formulations:

The present invention is drawn to non-aqueous formulations of peptide compounds in non-aqueous protic solvent which are stable for prolonged periods of time at elevated temperatures. Standard dilute aqueous peptide and protein formulations require manipulation of buffer type, ionic strength, pH and excipients (e.g., EDTA and ascorbic acid) to achieve stability. In contrast, the claimed formulations achieve stabilization of peptide compounds by the use of non-aqueous protic solvents. In particular, stability of high concentrations (at least about 10% w/w) of compound has been provided by the formulation of the present invention.

Examples of peptides and peptide compounds which may be formulated using the present invention include those peptides which have biological activity or which may be used to treat a disease or other pathological condition. They include, but are not limited to adrenocorticotropic hormone, angiotensin I and II, atrial natriuretic peptide, bombesin, bradykinin, calcitonin, cerebellin, dynorphin A, alpha and beta endorphin, endothelin, enkephalin, epidermal growth factor, fertirelin, follicular gonadotropin releasing peptide, galanin, glucagon, gonadorelin, gonadotropin, goserelin, growth hormone releasing peptide, histrelin, insulin, leuprolide, LHRH, motilin, nafarelin, neurotensin, oxytocin, somatostatin, substance P, tumor necrosis factor, triptorelin, and vasopressin. Analogs, derivatives, antagonists, agonists and pharmaceutically acceptable salts of the above may also be used.

The peptide compounds useful in the formulations and methods of the present invention can be used in the form of a salt, preferably a pharmaceutically acceptable salt. Useful salts are known to those of skill in the art and include salts with inorganic adds, organic acids, inorganic bases or organic bases. Preferred salts are acetate salts.

Peptides and peptide compounds which are readily soluble in non-aqueous protic solvents are preferred for use in the present invention. One of skill in the art can easily determine which compounds will be useful on the basis of their solubility, i.e., the compound must be soluble in the particular non-aqueous protic solvent to at least an acceptable amount, which may be a pharmaceutically effective amount. Preferred solubilities are at least about 10% (w/w). Particularly preferred peptide compounds are LHRH-related compounds, including leuprolide and leuprolide acetate.

The proportion of peptide may vary depending on the compound, the condition to be treated, the solubility of the compound, the expected dose and the duration of administration. (See, for example, The Pharmacological Basis of Therapeutics, Gilman et al., 7th ed. (1985) and Pharmaceutical Sciences, Remington, 18th ed. (1990), the disclosures of which are incorporated herein by reference.) The concentration of peptide in high concentration formulations may range from at least about 10% (w/w) to the maximum solubility of the compound. A referred range is from about 20 to about 60% (w/w). The currently more preferred range is from about 30 to about 50% (w/w) and a most preferred range is about 35 to about 45% (w/w).

Generally, the stable formulations of the present invention may be prepared by simply dissolving the desired amount of the desired peptide compound in the selected non-aqueous protic solvent. We have found that, for polymeric solvents such as PEG, solubility tends to be inversely proportional to the molecular weight of the solvent. Preferred non-aqueous protic solvents include propylene glycol (PG), polyethylene glycol (PEG), methoxypropylene glycol (MPEG), glycerol and polyvinylpyrrolidone (PVP).

It is known to those of skill in the art that water, buffer, solubilizers such as non-ionic surfactants, excipients such as EDTA and preservatives such as benzyl alcohols, methyl or propyl parabens may beneficially be added to pharmaceutical peptide formulations. (See, for example, Pharmaceutical Sciences, Remington, 18th ed. (1990).) Such agents may optionally be added to the claimed formulations.

C. Methodology:

We have found that stable non-aqueous formulations of peptide compounds may be prepared by dissolving the peptide compound to be formulated in non-aqueous protic solvents.

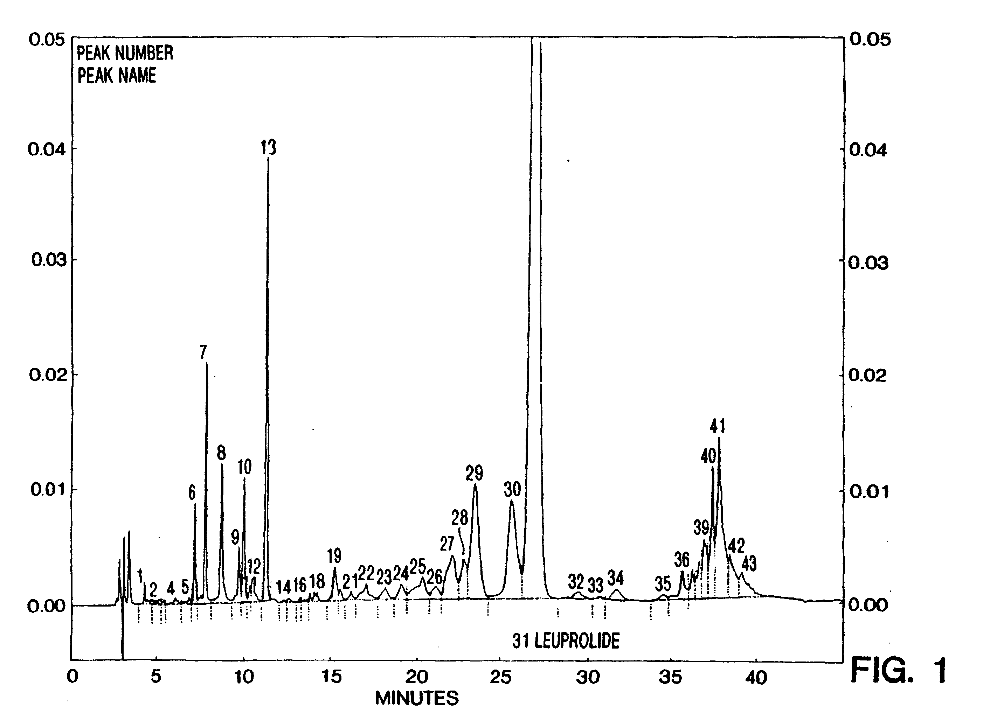

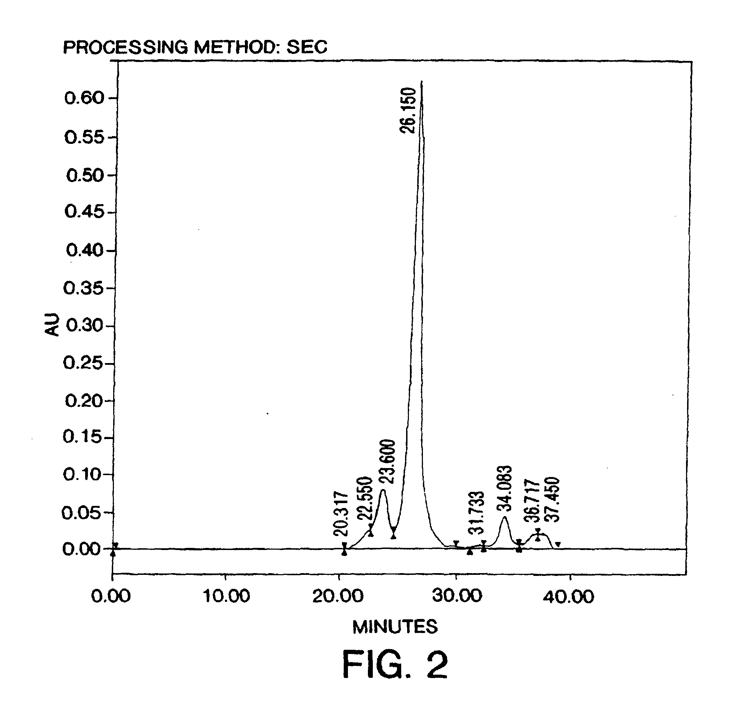

We have tested these peptide compound formulations, specifically formulations of the LHRH-related compound leuprolide, for stability by subjecting them to accelerated aging at elevated temperature and measuring the chemical and physical stability of the formulations. Results of these studies (shown, for example, in Table III and Figures 1, 2, 6 and 7) demonstrate that these formulations were stable at conditions that approximate or exceed storage for one year at 37°C.

We have also tested peptide compound formulations prepared as described herein for stability after 2.5 megarad gamma irradiation. Results, shown in Table IV, show that these formulations remained chemically and physically stable after such irradiation. Formulations subjected to electron beam irradiation were also found to be stable.

As shown in Table I, we have tested a wide variety of peptide formulations, specifically leuprolide, goserelin, LHRH, bradykinin, insulin and trypsinogen, for stability by dissolving (or attempting to dissolve) them in water, then subjecting them to accelerated aging at elevated temperatures. The stability of the formulations was measured. Results are presented in Table I as half-life at 37°C assuming an $E_a$ = 22.2 kcal/mole. A wide range of the peptides tested were soluble in the non-aqueous protic solvents tested and remained stable under the test conditions. The solubility of a particular peptide in water and the stability of the resulting solution are easily determined using routine procedures known to those of ordinary skill in the art.

Table I:

| Stability of Peptides in Non-Aqueous Protic Solvents | |
| --- | --- |
| FORMULATION | HALF-LIFE* (Temperature) |
| 40% Leuprolide in PG | 5.2 years (37°C) |
| 40% Goserelin in PG | 6.2 years (80°C) |
| 20% LHRH in PG | 1.2 years (65°C) |
| 20% Bradykinin in PG | 3.2 months (65°C) |
| 20% Insulin in PG | degraded w/in 24 hours (65°C) |

Table I: (continued)

| Stability of Peptides in Non-Aqueous Protic Solvents | |
|---|---|
| FORMULATION | HALF-LIFE* (Temperature) |
| 40% Trypsinogen in PG | insoluble |
| 40% Trypsinogen in PEG | insoluble |
| 20% Trypsinogen in PEG | 7.7 months (65°C) |
| *Half-life at 37°C assuming $E_a$ = 22.2 kcal/mole. | |

Formulations of 40% leuprolide in propylene glycol stored for six months at 37°C showed linear degradation as measured by overall loss of peptide from the solution. Analysis of these data gave an activation energy ($E_a$) of 16.6 kcal/mole and a $t_{90}$ of 9.6 months, showing stability of these formulations at elevated temperatures.

We have also unexpectedly found that certain peptide formulations of the present invention are bacteriostatic (i.e., inhibit bacterial growth), bactericidal (i.e., cause the death of bacteria), and sporicidal (i.e., kill spores). In particular, leuprolide formulations of 50-400 mg/ml exhibited bacteriostatic, bactericidal and sporicidal activity. The stability of the samples was unaffected by spiking with bacteria, indicating that the enzymes released from the killed and lysed bacteria did not adversely affect the stability of the product. This demonstrates that these formulations were not conducive to enzymatic activity.

Some peptides, for example calcitonin and leuprolide, are known to be physically unstable, exhibiting aggregation, gelation and fibrillation when formulated in solution in non-aqueous protic solvents as well as in aqueous solution. For example, leuprolide can be induced to gel by increasing peptide concentration, introduction of salts or gentle agitation. Improving physical stability can allow for easier parenteral administration, including administration using implantable drug delivery systems.

It has unexpectedly been found that adding polar aprotic solvents such as DMSO to non-aqueous protic solvent formulations of certain peptides, such as leuprolide, goserelin and calcitonin, prevents gelation of the formulation. This is apparently because non-aqueous polar aprotic solvents cause peptides to form a random coil/alpha helix conformation that does not refold into a beta sheet structure and, therefore, does not gel. Thus, these solvents have an anti-gellant effect.

Additionally, the stability of liquid and gelled (by agitation) leuprolide formulations in the non-aqueous protic solvent PG (370 mg/ml) was studied in vitro at 37°C and in vivo in rats, respectively. Results are presented in Table II, and show that the both gelled and liquid formulations remained stable over a period of 12 weeks.

Table II:

| Stability Studies of Liquid and Gelled Leuprolide Formulations in PG | | | |
|---|---|---|---|
| STUDY | TIME (weeks) | LIQUID (% remaining) | GELLED (% remaining) |
| Long Term Stab | 6 | 98.50 | |
| Long Term Stab | 12 | 98.00 | |
| Rat | 6 | | 97.40 |
| Rat | 12 | | 95.90 |

A major aspect of the invention is that non-aqueous solutions containing peptide compounds in non-aqueous protic solvents are stable at high temperatures for long periods of time. Such formulations are stable even when high concentrations are used. Thus, these formulations are advantageous in that they may be stored for long time periods at or above room temperature. They are also suitable for use in implantable delivery devices.

DISCLOSURE OF EXAMPLES OF THE INVENTION

The following methods were used to perform the studies in the Examples that follow.

1. Preparing leuprolide acetate solutions

Leuprolide acetate (obtained, for example, from Mallinckrodt, St. Louis, Missouri) was weighed and dissolved using heat (80°C), swirling, agitation and/or centrifugation as needed, in vehicle (PG, PEG, MPEG, PG/$H_2$O, PG/$H_2$O, PEG/PG, MPEG/$H_2$O, or PG with EDTA) at the appropriate concentration (w/w). Unless otherwise noted the term PEG means PEG 300. The term dry PG refers to PG formulations prepared in a low moisture environment (i.e., dry $N_2$ atmosphere).

Unless otherwise noted, leuprolide free base content was calculated from certificate of analysis potency values to be 37% free base. This was 40% leuprolide acetate, except as noted.

2. Preparation of reservoirs

The reservoirs of implantable drug delivery devices (as disclosed in U.S. Patent Application Serial No. 08/595,761, incorporated herein by reference) were filled with the appropriate leuprolide acetate solution. The filled devices then underwent stability testing. The formulation was filled into titanium or polymer reservoirs with a polymeric plug blocking each end. The filled reservoir was then sealed in a polyfoil bag and placed in a stability testing oven.

It should be noted that the formulations inside the reservoirs of these devices are completely isolated from the outside environment.

3. Reverse Phase-HPLC (RP-HPLC)

All stability samples were analyzed for leuprolide concentration and % peak area using a gradient elution reversed-phase HPLC assay with a refrigerated autosampler (4°C) to minimize sample degradation. The chromatographic conditions used are listed below.

| RP-HPLC Chromatographic Conditions | | | | | | | | | |
|---|---|---|---|---|---|---|---|---|---|
| Description | Parameter | | | | | | | | |
| Column | HaiSil C18, 4.6 X 250mm, S/N 5103051 | | | | | | | | |
| Flow Rate | 0.8 mL min$^{-1}$ | | | | | | | | |
| Injection Volume | 20 μL | | | | | | | | |
| Detection | 210 nm | | | | | | | | |
| Leuprolide Retention Time | Between 25-30 minutes | | | | | | | | |
| Mobile Phase | A = 100 mM Sodium Phosphate, pH 3.0 B = 90% Acetonitrile/Water | | | | | | | | |
| Gradient | Minutes | 0 | 5 | 25 | 40 | 41 | 46 | 46.1 | 50 |
| | %B | 15 | 26.5 | 26.5 | 65 | 85 | 85 | 15 | 15 |

Leuprolide standards (in water) at 4 to 6 different concentration levels, typically between 0.1 - 1.2 mg/mL, were run along with the stability samples. The stability samples were bracketed by the standard sets, with no more than 40 samples in between the standard sets. All peaks between the void volume and 45 minutes of the run were integrated. The integrated peak areas for the leuprolide standards were plotted as a function of the concentration. The leuprolide concentrations for the stability samples were then calculated using linear regression. The % peak areas for the leuprolide peak, the sum of all the peaks eluting before leuprolide (labeled "others"), and the sum of all the peaks eluting after leuprolide (labeled "aggregates") were also recorded and plotted as a function of the sample time points.

4. Size Exclusion Chromatography (SEC)

Selected stability samples were analyzed for % peak area and molecular weights using an isocratic solution SEC assay with a refrigerated autosampler (4°C). The chromatographic conditions used are listed below.

| SEC Chromatographic Conditions | |
|---|---|
| Description | Parameter |
| Column | Pharmacia Peptide, HR 10/30,10 X 300 mm |
| Flow Rate | 0.5 mL min$^{-1}$ |
| Injection Volume | 20 μL |
| Detection | 210 nm |
| Leuprolide Retention Time | Approximately 25 minutes |
| Mobile Phase | 100 mM Ammonium Phosphate, pH 2.0, 200 mM Sodium Chloride, 30% Acetonitrile |

The void volume and total volume for the size exclusion column was needed for the calculation of the molecular weights. The Bio-Rad high molecular weight standard and 0.1% acetone were used to determine the void volume and total volume respectively. The retention times for the first peak in the Bio-Rad standard and the acetone peak were recorded and converted to volume units using the equations below. Since these values are constant for a particular SEC column and HPLC system, the void and total volumes were redetermined whenever changes to the SEC column or HPLC system were made. A standard run was then made followed by the stability samples. The standard mixture contained approximately 0.2 mg/mL of the following peptides: Bursin (MW=449), WLFR peptide (MW=619), Angiotensin (MW=1181), GRF (MW=5108), and Cytochrome C (MW=12394). These standards were chosen because they bracketed leuprolide molecular weight and all had basic pI (9.8 - 11.0), similar to leuprolide.

The % peak areas were recorded for all the peaks. The molecular weights for the species separated were calculated using the equations below.

$V_s$ = flow rate (mL/min) x sample peak retention time (min)
$V_o$ = flow rate (mL/min) x void volume peak retention time (min)
$V_t$ = flow rate (mL/min) x total volume peak retention time (min)

$$Kd = \frac{V_s - V_o}{V_t - V_o}$$

Where:

$V_s$ = standard or sample volume
$V_o$ = void volume
$V_t$ = total volume

$V_s$ was calculated to each peptide standard peak. Kd for each peptide standard was then calculated using the values for $V_t$ and $V_o$ determined earlier. The linear regression line from the plot of logMW vs. $Kd^{-1}$ was used to determine the molecular weights for each peak in the stability sample. The % peak areas for the stability samples were also recorded.

5. Instrumentation and Materials

The instrumentation and materials used for RP-HPLC and SEC were as follows:

Waters Millennium HPLC system consisting of 717 autosampler, 626 pump, 6000S controller, 900 photodiode array detector, and 414 refractive index detector (Waters Chromatography, Milford, MA)
HPLC vials, for 48-position and 96-position (Waters Chromatography, Milford, MA)
HaiSil C18, 120 A, 5 μm4.6 x 250 mm HPLC column (Higgins Analytical, Mountain View, CA)
Pharmacia Peptide, HR 10/30 SEC column (Pharmacia Biotech, Piscataway, NJ)

6. Purity

Stability samples were analyzed using RP-HPLC. The area under the curve for the leuprolide peak divided by the sum of the areas under the curve of all peaks gave % purity. [It should be noted that the data for % concentration presented with the % purity data (Examples 6, 8, 9 and 10) are inconclusive. The analysis methods used to determine % concentration in these experiments were unreliable.]

The following examples are offered to illustrate this invention and are not meant to be construed in any way as limiting the scope of this invention.

## EXAMPLE 1

Accelerated Stability Studies of Leuprolide Acetate Formulations

Formulations of 40% (w/w) leuprolide acetate (equivalent to 37% leuprolide free base) in vehicle were prepared as described above and used to fill the reservoirs of implantable drug delivery devices, also as described above. Some reservoirs were made of polymer materials, while some were titanium.

The filled devices were subjected to accelerated aging by storing them at elevated temperatures (80-88°C) for seven days in an incubator (Precision Scientific or Thelco). This is equivalent to about six months at 37°C or about one year at room temperature (25°C), assuming an activation energy ($E_a$) of 16.6 kcal/mole.

The samples were analyzed using RP-HPLC and SEC as described above to determine the chemical and physical stability of the aged formulations.

Results, presented in Table III, demonstrate that these formulations were able to maintain the stability of the LHRH-related compound leuprolide. In each case, at least 65% leuprolide was retained.

Table III

| Stability of Leuprolide Acetate Non-Aqueous Protic Formulations After 7 Days at Elevated Temperatures | | | |
|---|---|---|---|
| Temperature (°C) | Reservoir Material | Formulation | % Leuprolide at Day 7 |
| 88 | Polymer | 40% in PG | 70 |
| 88 | Polymer | 40% in PG/$H_2$O (70/30) | 73 |
| 88 | Polymer | 40% in PEG/$H_2$O (90/10) | 77 |
| 88 | Titanium | 40% in PG | 87 |
| 88 | Polymer | 20% in PEG/PG(50/50) | 74 |
| 88 | Polymer | 20% in PEG/$H_2$O(88/12) | 68 |
| 80 | Polymer | 40% in PG | 74 |
| 80 | Titanium | 40% in PG | 80 |
| 80 | Titanium | 40% in PEG/$H_2$O(90/10) | 86 |
| 80 | Titanium | 40% in PG | 87 |
| 80 | Titanium | 40% in PG | 80 |
| 80 | Titanium | 40% in 1%EDTA in PG | 80 |
| 80 | Polymer | 40% in MPEG 350/$H_2$O(50/50) | 83 |
| 80 | Titanium | 40% in dry PG | 76 |

**EXAMPLE 2**

Stability Studies of Irradiated Leuprolide Acetate Formulations

Formulations of 40% (w/w) as received leuprolide acetate (equivalent to 37% leuprolide free base) in PG were prepared as described above and used to fill the reservoirs of drug delivery devices, also as described above. All reservoirs were made of polymer materials.

The filled devices were subjected to 2.5 megarad gamma irradiation. Samples were shipped to Sterigenics (Tustin, California) and gamma irradiated (Cobalt 60) in batch mode. Samples labeled "cold" were shipped and irradiated on dry ice. Samples were then subjected to accelerated aging as in Example 1. Samples were taken at day 0 and day 7, and analyzed using RP-HPLC and SEC as described above to determine the chemical and physical stability of the irradiated formulations.

Results, presented in Table IV, demonstrate that these leuprolide acetate formulations were stable after irradiation. In every case, at least 65% leuprolide was retained, with low levels of aggregate formation.

EP 0 909 175 B1

## Table IV

### Stability of 40% (w/w) Leuprolide Acetate Protic Formulations After 2.5 Megarad Gamma Irradiation in Polymer Reservoirs

| Formulation | Irradiation | % Leuprolide at Day 7 (RP-HPLC) | SEC | | | |
|---|---|---|---|---|---|---|
| | | | Day 0 | | Day 7 | |
| | | | % monomer | % dimer/trimer | % monomer | % dimer/trimer |
| 40% in PG | Yes | 88 | 97.4 | 0.9 | 94.5 | 3.7 |
| 40% in PG | No | 75 | 98.8 | 0.03 | 91.9 | 5.9 |
| 40% in PG | Cold | 75 | 98.3 | 0.2 | 92.2 | 5.6 |

## EXAMPLE 3

Solubility Studies of Leuprolide Acetate in PG

Leuprolide acetate formulations in PG were prepared as described above. Formulations were heated at 80°C to accelerate the dissolution of leuprolide in PG. The data are presented in Table V below.

Table V

| % Leuprolide in PG | | | |
|---|---|---|---|
| Wt. Leuprolide Acetate (mg) | Wt. PG (mg) | Total Wt. | % Leuprolide Acetate |
| 148.6 | 225.7 | 374.3 | 39.70 |
| 154 | 183.7 | 337.7 | 45.60 |
| 146.8 | 147.2 | 294 | 49.93 |

## EXAMPLE 4

Long Term Accelerated Stability Studies of Leuprolide Acetate in PG

Solutions of 40 % leuprolide acetate (w/w) in PG were prepared, loaded into reservoirs, stored for two months at 80°C and analyzed as described above. Results, shown in Figures 1 (RP-HPLC) and 2 (SEC) show that 55.9% leuprolide was recovered, with only 37.2% chemical degradation and 15.2% physical aggregation after the two month period. These formulations were stable (as defined above) after seven days at 80°C, which corresponds to two months at 37°C.

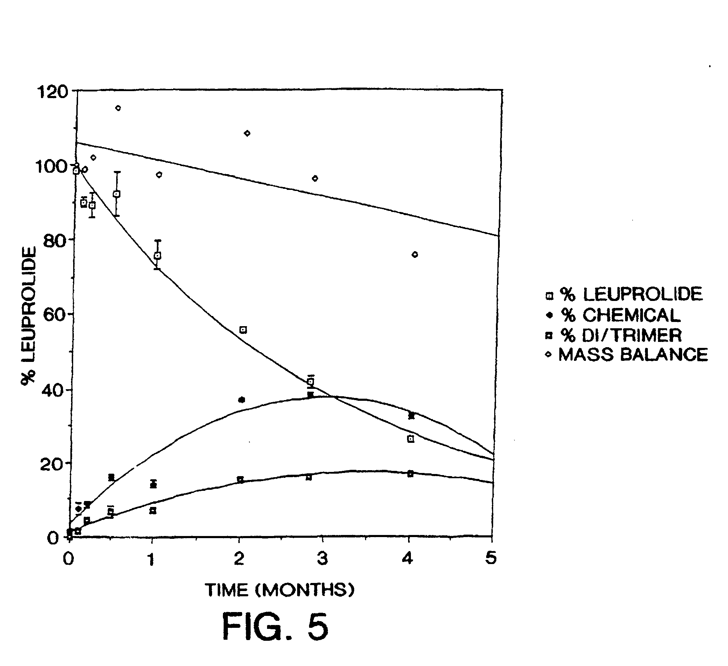

Solutions of 40% leuprolide acetate (w/w) in PG were prepared, loaded into reservoirs, stored at 80°C for four months and analyzed using RP-HPLC as described above. Figure 5 is a plot of leuprolide, and its chemical and physical degradation products recovered over the four month time period. The sum of these three elements is also presented as mass balance. The results show that we can account for all the peptide material as either intact leuprolide or a degradation species, indicating that stability studies are not missing an unknown degradation process or product.

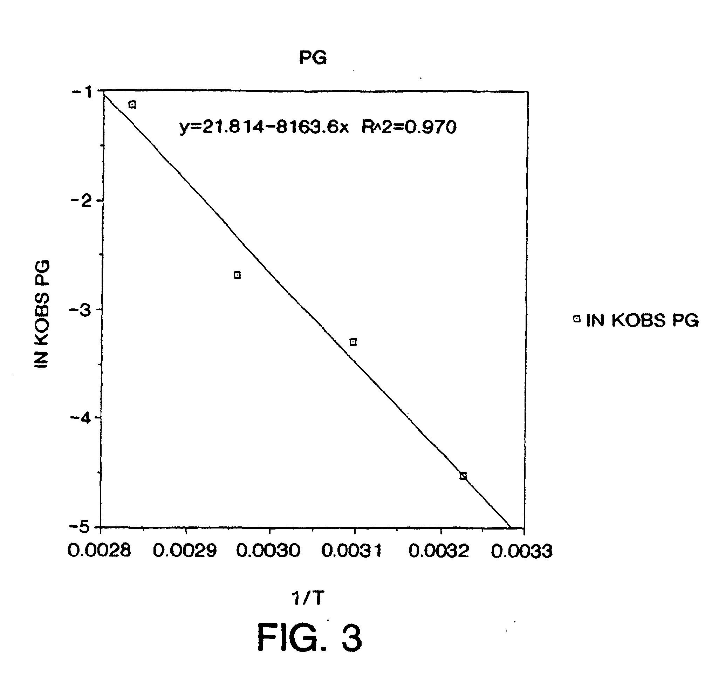

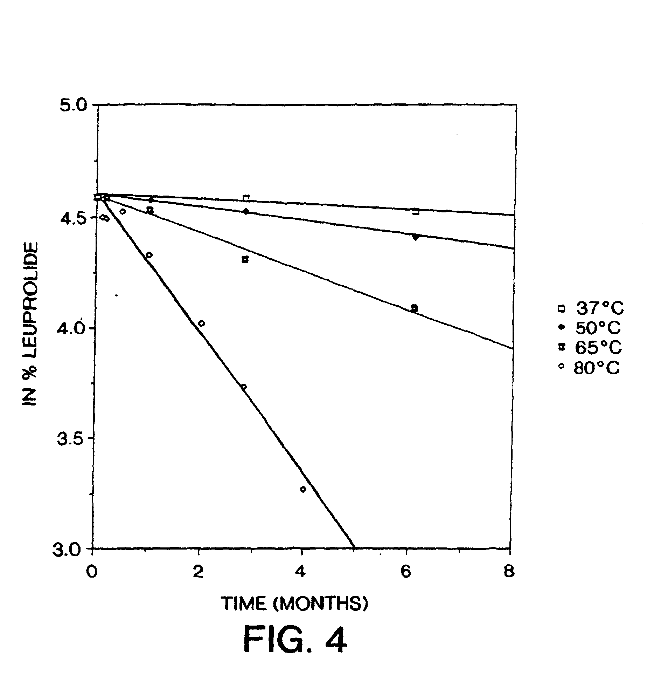

Solutions of 40% leuprolide acetate (w/w) in PG were prepared, loaded into reservoirs, stored at 37°C, 50°C, 65°C or 80°C for four to six months and analyzed using RP-HPLC as described above. Results, presented in Figure 4, show that leuprolide degradation fits pseudo first order kinetics. Furthermore, as discussed below, Figure 3 indicates that leuprolide in PG degradation fits linear Arrhenius kinetics. Therefore, accelerated stability studies are a valid technique for assessing the stability of leuprolide and extrapolating back to 37°C.

Solutions of 40% leuprolide acetate (w/w) in PG were prepared, loaded into reservoirs, stored at 37°C, 50°C, 65°C or 80°C and analyzed using RP-HPLC as described above. Results were calculated as described in Physical Pharmacy: Physical Chemical Principles in the Pharmaceutical Sciences, 3rd ed., Martin et al., Chapter 14 (1983) and showed the $E_a$ of these solutions to be 16.6 kcal/mole with a $t_{90}$ of 9.6 months at 37°C. The data are shown below and an Arrhenius plot of the data is presented in Figure 3.

| PG | | |
|---|---|---|
| °C | Kobs (months$^{-1}$) | $t_{1/2}$ (months) |
| 37 | $1.12 \times 10^{-2}$ | 61.6 |
| 50 | $3.13 \times 10^{-2}$ | 22.2 |
| 65 | $8.64 \times 10^{-2}$ | 8.0 |
| 80 | 0.322 | 2.4 |

$E_a$ = 16.6 kcal/mole

**EXAMPLE 5**

Long Term Stability Studies of Leuprolide Acetate in PG

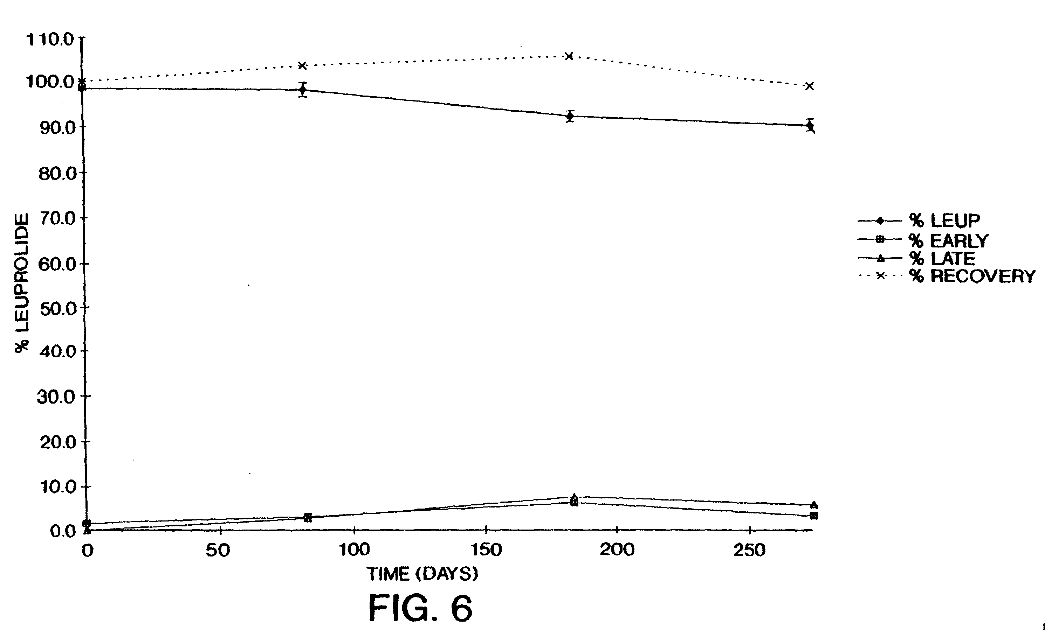

The chemical stability of 40% leuprolide acetate solutions prepared and analyzed as described above is presented in Figure 6. After nine months at 37°C more than 90% (90.1%) leuprolide was present, with less than 5% (3.1%) chemical degradation products (shown as "early") and less that 10 % (5.6%) physical aggregation (shown as "late"), based on RP-HPLC data but in good agreement with SEC data, being formed.

**EXAMPLE 6**

Long Term Stability Studies of Leuprolide Acetate in PG/Acetate Buffer

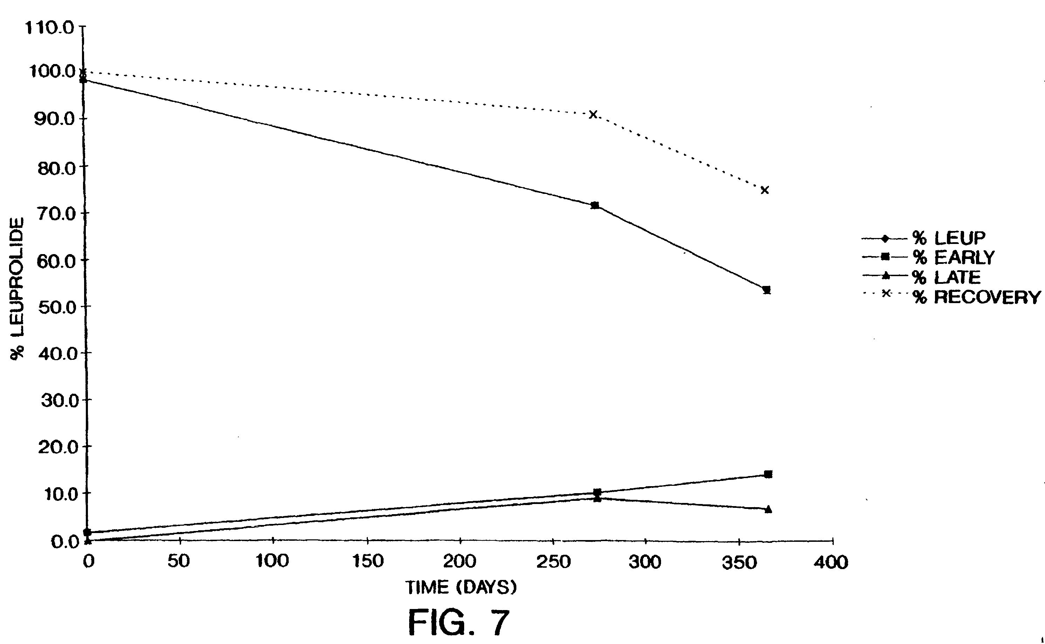

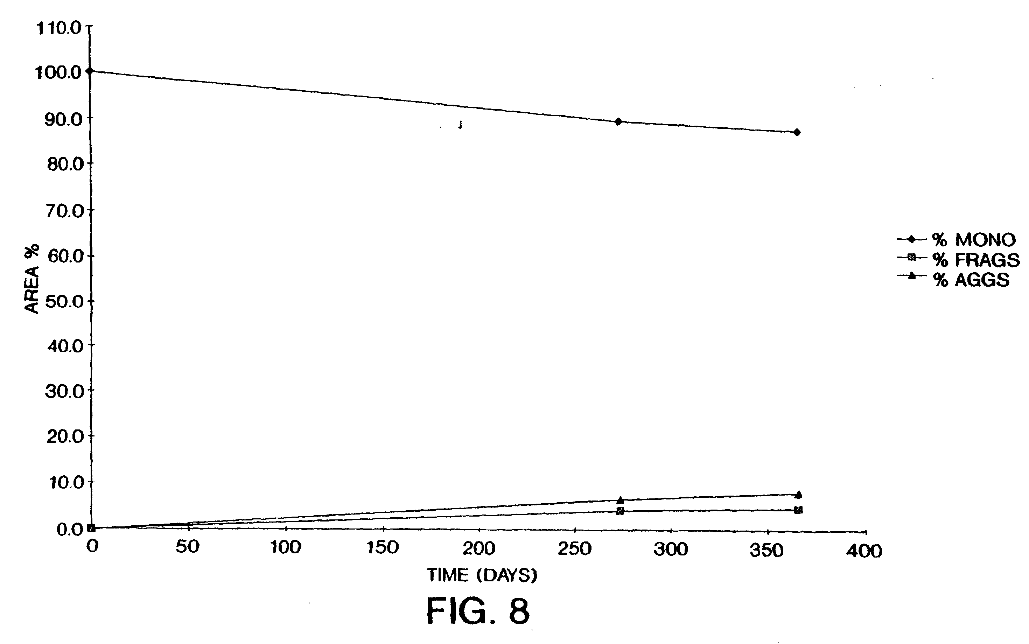

Solutions of 30% leuprolide acetates (w/w) in PG/acetate buffer (pH 5.0, 0.0282M) (30:70) were prepared as described above then loaded into glass ampules, irradiated as described above and stored at 37°C for one year. Analysis (as described above) by RP-HPLC (Figure 7) and SEC (Figure 8) showed that these formulations were stable. After nine months, RP-HPLC showed that over 70% chemically active leuprolide was present in the formulations. SEC results showed that 90% physically stable leuprolide was present after 9 months at 37°C.

**EXAMPLE 7**

Long Term Accelerated Stability Studies of Leuprolide Acetate in PG/Water

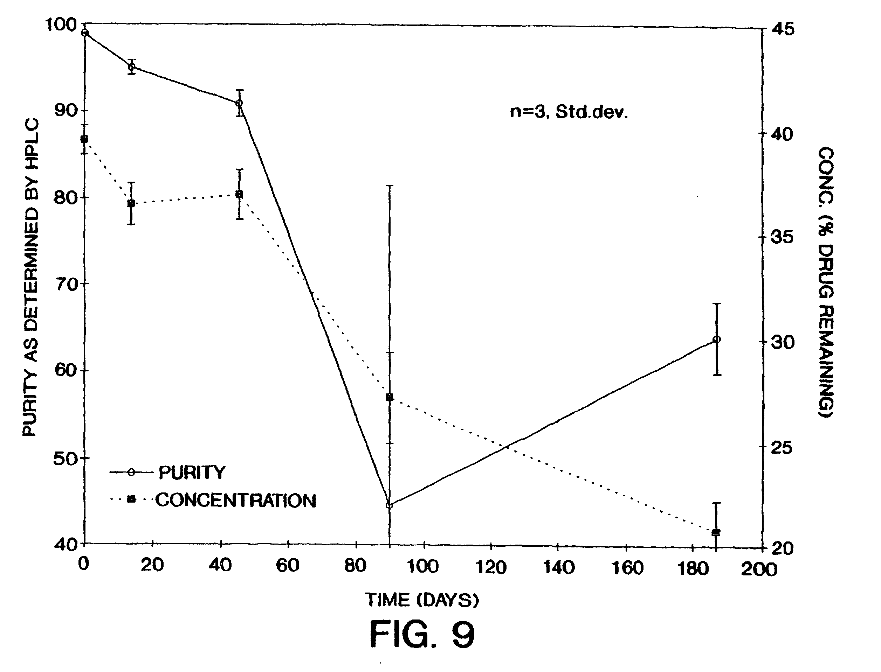

Formulations of 40% leuprolide acetate (w/w) in PG/water with preservatives (30:70) were prepared by mixing 0.18% methyl paraben and 0.025% propylparaben with water, preparing a 30:70 PG/water with preservative solution and dissolving the leuprolide acetate in this solution as described above. Formulations were loaded into glass ampules, then irradiated and stored at 60°C as described above.
Purity was assayed over a six month period as described above. Results are presented in Figure 9. These data show that these formulations had purity of over 90% at 45 days and about 65% at six months. The 90 day data point showed a very high standard deviation.

**EXAMPLE 8**

Long Term Stability Studies of Leuprolide Acetate in PG/Water

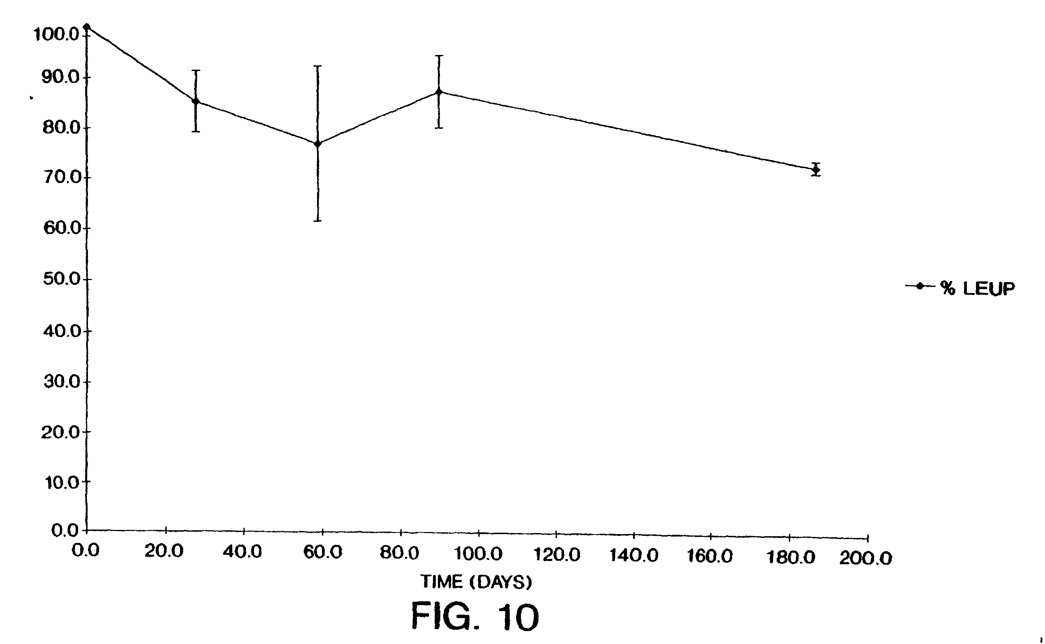

Formulations of 40% leuprolide acetate (w/w) in PG/water (30:70) were prepared as described above, loaded into glass ampules, irradiated and stored at 37°C for six months as described above, then assayed using HPLC.
Results, presented in Figure 10, showed that over 70% leuprolide remained after six months.

**EXAMPLE 9**

Accelerated Stability Studies of Goserelin in PEG 600/Acetate Buffer

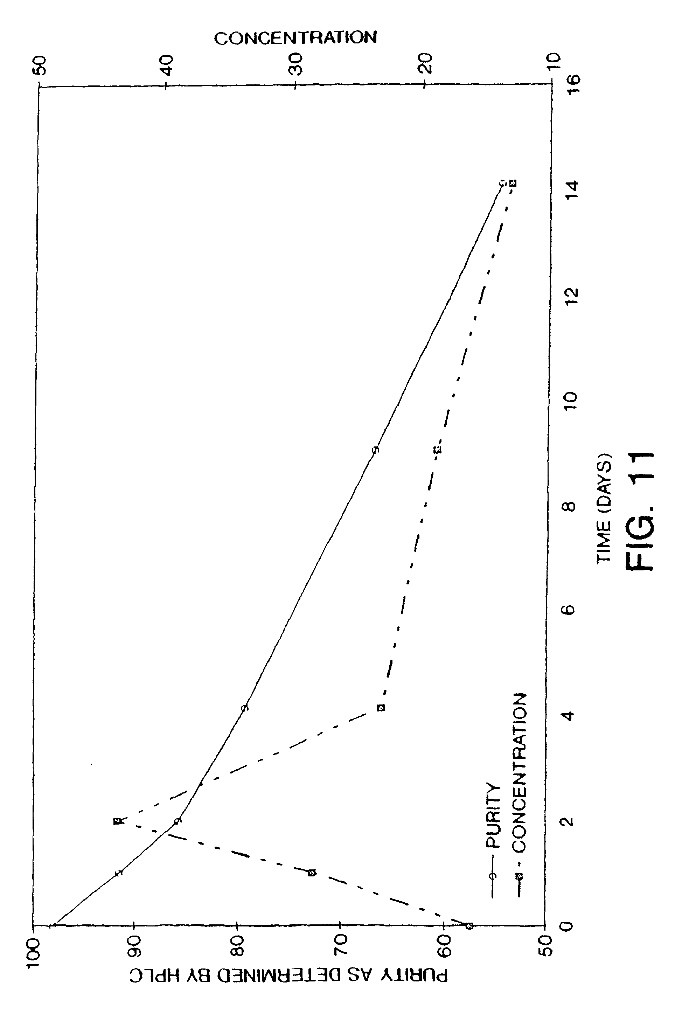

Formulations of 30% goserelin (w/w) in PEG 600/acetate buffer (30:70), prepared as described above for leuprolide acetate, were stored in glass ampules for 14 days at 80°C and analyzed for purity as described above.
Results in Figure 11 show that after nine days over 65% goserelin remained.

**EXAMPLE 10**

Stability Studies of Goserelin Formulations

Formulations of 40-45% (w/w) goserelin in either PEG 600 or PG/acetate buffer (30:70) were prepared as described above and placed in polymeric containers. The containers were stored at 37°C for one month in an incubator. The samples were analyzed using RP-HPLC to determine the chemical stability of the aged formulations.
Results, presented below, demonstrate that these formulations were able to maintain the stability of the LHRH-related compound goserelin. In each case, at least 98% goserelin was retained, as indicated by the purity data.

| DRUG | VEHICLE | % PURITY | % CONCENTRATION |
|------|---------|----------|------------------|
| goserelin | PEG 600 | 99.3 | 23.6 |
| goserelin | PG/Acetate Buffer(30:70) | 98.2 | 49.7 |

## EXAMPLE 11

Stability Studies of Nafarelin Formulations

Formulations of 15% (w/w) nafarelin in either PEG 600 or propylene glycol were prepared as described above for leuprolide and placed in polymeric containers.

The containers were stored at 37°C for one month in an incubator.

The samples were analyzed using RP-HPLC to determine the chemical stability of the aged formulations.

Results, presented below, demonstrated that these formulations were able to maintain the stability of the LHRH-related compound nafarelin. In each case, at least 99% nafarelin was retained, as indicated by the purity data.

| DRUG | VEHICLE | % PURITY | % CONCENTRATION |
|------|---------|----------|------------------|
| nafarelin | PEG 600 | 99.4 | 15.8 |
| nafarelin | PG | 99.4 | 12.9 |

Modification of the above-described modes of carrying out various embodiments of this invention will be apparent to those of skill in the art following the teachings of this invention as set forth herein. The examples described above are not limiting, but are merely exemplary of this invention, the scope of which is defined by the following claims.

## Claims

1. A stable non-aqueous formulation for administration to a patient, comprising:

   a) at least one peptide compound; and
   b) a solvent for the peptide compound, which solvent consists of at least one non-aqueous protic solvent in which said peptide compound has a solubility of at least 10% (w/w) and in which the peptide compound is dissolved,

   wherein said formulation is stable at 37°C for at least 2 months, so that at least 65% chemically and physically stable peptide compound remains after two months at 37°C.

2. A method for preparing the stable non-aqueous formulation of claim 1 comprising providing at least one peptide compound and selecting a solvent for the peptide compound which solvent consists of at least one non-aqueous protic solvent in which said peptide compound has a solubility of at least 10% (w/w), and dissolving said peptide compound in the non-aqueous protic solvent.

3. The formulation of claim 1 or the method of claim 2 wherein there is at least 10% (w/w) peptide compound.

4. The formulation of claim 1 or the method of claim 2 wherein there is at least 30% (w/w) peptide compound.

5. The formulation or method of any preceding claim wherein said peptide compound is an LHRH-related compound.

6. The formulation or method of claim 5 wherein said peptide compound is selected from the group consisting of leuprolide, LHRH, nafarelin and goserelin.

7. The formulation or method of any preceding claim wherein the formulation is adapted for use in an implantable drug delivery device.

8. The formulation or method of any preceding claim wherein said at least one non-aqueous protic solvent is selected from the group consisting of PG, PEG and glycerol.

9. The formulation or method of any preceding claim wherein the formulation forms a gel.

10. The formulation or method of any of claims 1 to 8 wherein the formulation is further provided with at least one non-aqueous polar aprotic solvent.

11. The formulation or method of claim 10 wherein said polar aprotic solvent is DMSO or DMF.

12. The formulation or method of any preceding claim wherein the formulation is further provided with at least one selected from the group consisting of an excipient, a surfactant, a solubilizer, and a preservative.

13. The formulation or method of claim 1 or claim 2 which consists essentially of 30% to 50% (w/w) of the LHRH-related compound leuprolide acetate in PG or PEG or a mixture thereof.

14. The method of claim 2 to produce a formulation which is stable after irradiation.

15. The method of claim 2 to produce a formulation which is stable at 37°C for at least one year.

16. The method of claim 2 to produce a stable non-aqueous formulation which exhibits bacteriostatic, bactericidal or sporicidal activity.

17. The method of claim 16 wherein the peptide is leuprolide present in the formulation in a concentration of 50 to 400 mg/ml.

18. The method of claim 10 or claim 11 to form a non-gelling formulation.

19. The method of claim 18 wherein the peptide is selected from leuprolide, goserelin and calcitonin.

**Patentansprüche**

1. Stabile, nicht-wäßrige Formulierung zur Verabreichung an einen Patienten, bestehend aus:

   a) mindestens einer Peptidverbindung, und

   b) einem Lösungsmittel für die Peptidverbindung, wobei das Lösungsmittel aus mindestens einem nicht-wäßrigen protischen Lösungsmittel besteht, in dem die Peptidverbindung eine Löslichkeit von wenigstens 10% (w/w) hat und in dem die Peptidverbindung gelöst ist,

   wobei die Formulierung bei 37°C für mindestens zwei Monate stabil ist, so daß nach zwei Monaten bei 37°C mindestens 65% einer chemisch und physikalisch stabilen Peptidverbindung verbleiben.

2. Verfahren zum Herstellen der stabilen, nicht-wäßrigen Formulierung nach Anspruch 1, umfassend das Bereitstellen mindestens einer Peptidverbindung und das Auswählen eines Lösungsmittels für die Peptidverbindung, wobei das Lösungsmittel aus mindestens einem nicht-wäßrigen, protischen Lösungsmittel besteht, in dem die Peptidverbindung eine Löslichkeit von wenigstens 10% (w/w) hat, sowie das Auflösen der Peptidverbindung in dem nicht-wäßrigen, protischen Lösungsmittel.

3. Formulierung nach Anspruch 1 oder Verfahren nach Anspruch 2, wobei mindestens 10% (w/w) Peptidverbindung vorhanden sind.

4. Formulierung nach Anspruch 1 oder Verfahren nach Anspruch 2, wobei mindestens 30% (w/w) Peptidverbindung vorhanden sind.

5. Formulierung oder Verfahren nach einem der vorhergehenden Ansprüche, wobei die Peptidverbindung eine LHRH verwandte Verbindung ist.

6. Formulierung oder Verfahren nach Anspruch 5, wobei die Peptidverbindung aus der Gruppe bestehend aus Leuprolid, LHRH, Nafarelin und Goserelin ausgewählt wird.

7. Formulierung oder Verfahren nach einem der vorhergehenden Ansprüche, wobei sich die Formulierung zur Verwendung in einer implantierbaren Arzneimittelverabreichungsvorrichtung eignet.

8. Formulierung oder Verfahren nach einem der vorhergehenden Ansprüche, wobei das mindestens eine nicht-wäßrige, protische Lösungsmittel aus der Gruppe bestehend aus PG, PEG und Glycerin ausgewählt wird.

9. Formulierung oder Verfahren nach einem der vorhergehenden Ansprüche, wobei die Formulierung ein Gel bildet.

10. Formulierung oder Verfahren nach einem der Ansprüche 1 bis 8, wobei die Formulierung ferner mit mindestens einem nicht-wäßrigen, polaren, aprotischen Lösungsmittel versehen wird.

11. Formulierung oder Verfahren nach Anspruch 10, wobei das polare, aprotische Lösungsmittel DMSO oder DMF ist.

12. Formulierung oder Verfahren nach einem der vorhergehenden Ansprüche, wobei die Formulierung ferner mit mindestens einem Bestandteil versehen wird, der aus der Gruppe bestehend aus einem Träger, einem Surfactant, einem Lösungsvermittler und einem Konservierungsmittel ausgewählt wird.

13. Formulierung oder Verfahren nach Anspruch 1 bzw. Anspruch 2, im wesentlichen bestehend aus 30% bis 50% (w/w) der LHRH verwandten Verbindung Leuprolidacetat in PG oder PEG oder einer Mischung derselben.

14. Verfahren nach Anspruch 2 zur Herstellung einer Formulierung, die nach Bestrahlung stabil ist.

15. Verfahren nach Anspruch 2 zur Herstellung einer Formulierung, die bei 37°C für mindestens ein Jahr stabil ist.

16. Verfahren nach Anspruch 2 zur Herstellung einer stabilen, nicht-wäßrigen Formulierung, die eine bakteriostatische, bakterizide oder sporizide Wirkung aufweist.

17. Verfahren nach Anspruch 16, wobei das Peptid Leuprolid ist, das in der Formulierung in einer Konzentration von 50 bis 400 mg/ml vorhanden ist.

18. Verfahren nach Anspruch 10 oder 11 zum Bilden einer nicht-gelbildenden Formulierung.

19. Verfahren nach Anspruch 18, wobei das Peptid aus Leuprolid, Goserelin und Calcitonin ausgewählt wird.

**Revendications**

1. Formulation non aqueuse stable destinée à une administration à un patient, comprenant :

   a) au moins un composé peptidique ; et
   b) un solvant pour le composé peptidique, lequel solvant consiste en au moins un solvant protique non aqueux dans lequel ledit composé peptidique a une solubilité d'au moins 10% (m/m) et dans lequel le composé peptidique est dissous,

   dans laquelle ladite formulation est stable à 37°C pendant au moins 2 mois, de telle sorte qu'il reste au moins 65% de composé peptidique chimiquement et physiquement stable après deux mois à 37°C.

2. Méthode de préparation de la formulation non aqueuse stable selon la revendication 1 comprenant l'apport d'au moins un composé peptidique et la sélection d'un solvant pour le composé peptidique, lequel solvant consiste en au moins un solvant protique non aqueux dans lequel ledit composé peptidique a une solubilité d'au moins 10% (m/m), et la dissolution dudit composé peptidique dans le solvant protique non aqueux.

3. Formulation selon la revendication 1 ou la méthode selon la revendication 2 dans laquelle il y a au moins 10% (m/m) de composé peptidique.

4. Formulation selon la revendication 1 ou la méthode selon la revendication 2 dans laquelle il y a au moins 30% (m/m) de composé peptidique.

**5.** Formulation ou méthode selon l'une quelconque des revendications précédentes dans laquelle ledit composé peptidique est un composé apparenté à la LHRH.

**6.** Formulation ou méthode selon la revendication 5 dans laquelle ledit composé peptidique est choisi dans le groupe consistant en le leuprolide, la LHRH, la nafaréline et la goséréline.

**7.** Formulation ou méthode selon l'une quelconque des revendications précédentes dans laquelle la formulation est adaptée à une utilisation dans un dispositif de délivrance de médicaments implantable.

**8.** Formulation ou méthode selon l'une quelconque des revendications précédentes dans laquelle ledit au moins un solvant protique non aqueux est choisi dans le groupe consistant en le PG, le PEG et le glycérol.

**9.** Formulation ou méthode selon l'une quelconque des revendications précédentes dans laquelle la formulation forme un gel.

**10.** Formulation ou méthode selon l'une quelconque des revendications 1 à 8 dans laquelle la formulation est en outre pourvue d'au moins un solvant polaire aprotique non aqueux.

**11.** Formulation ou méthode selon la revendication 10 dans laquelle ledit solvant polaire aprotique est le DMSO ou le DMF.

**12.** Formulation ou méthode selon l'une quelconque des revendications précédentes dans laquelle la formulation est en outre pourvue d'au moins un élément choisi dans le groupe consistant en un excipient, un agent tensioactif, un agent solubilisant, et un conservateur.

**13.** Formulation ou méthode selon la revendication 1 ou la revendication 2 qui est essentiellement constituée de 30% à 50% (m/m) du composé apparenté à la LHRH acétate de leuprolide dans du PG ou du PEG ou un mélange de ceux-ci.

**14.** Méthode selon la revendication 2 pour produire une formulation qui est stable après irradiation.

**15.** Méthode selon la revendication 2 pour produire une formulation qui est stable à 37°C pendant au moins un an.

**16.** Méthode selon la revendication 2 pour préparer une formulation non aqueuse stable qui présente une activité bactériostatique, bactéricide ou sporicide.

**17.** Méthode selon la revendication 16 dans laquelle le peptide est le leuprolide présent dans la formulation à une concentration de 50 à 400 mg/ml.

**18.** Méthode selon la revendication 10 ou la revendication 11 pour former une formulation non gélifiante.

**19.** Méthode selon la revendication 18 dans laquelle le peptide est choisi parmi le leuprolide, la goséréline et la calcitonine.

FIG. 1

EP 0 909 175 B1

FIG. 2

PG

$y=21.814-8163.6x$   $R^2=0.970$

□ IN KOBS PG

1/T

# FIG. 3

FIG. 4

FIG. 5

FIG. 6

EP 0 909 175 B1

FIG. 7

FIG. 8

EP 0 909 175 B1

FIG. 9

EP 0 909 175 B1

FIG. 10

EP 0 909 175 B1

FIG. 11

EP 0 909 175 B1